# FASCICULE DE BREVET EUROPEEN

(11) **EP 0 760 241 B1**
(45) Date de publication et mention de la délivrance du brevet: **17.06.1998**
(21) Numéro de dépôt: 96401845.1
(22) Date de dépôt: 29.08.1996
(51) Int. Cl.: A61K 31/60

(54) **Utilisation de la sulfasalazine et/ou de son métabolite actif pour la fabrication d'un médicament utile dans le traitement de l'insuffisance veineuse et des ulcères veineux**
Verwendung von Sulfasalazin und/oder dessen aktiven Metaboliten zur Herstellung eines Arzneimittels zur Behandlung von venöser Insuffizienz und venösen Ulcera
Use of sulfasalazine and/or its active metabolite for the manufacture of a medicament for the treatment of venous insufficiency and venous ulcers

(30) Priorité: 01.09.1995 FR 9510291
(43) Date de publication de la demande: 05.03.1997
(73) Titulaire: SYNTHELABO, 92350 Le Plessis Robinson (FR)
(72) Inventeur: Berry, Christopher, 94500 Champigny sur Marne (FR)
(74) Mandataire: Thouret-Lemaitre, Elisabeth

(56) Documents cités:
- EP-A- 0 352 826
- WO-A-86/03199
- DIG.DIS.SCI., vol. 40, no. 1, Janvier 1995, pages 219-225, XP000569608 N.POOLEY ET AL.: "Up-Regulation of E-Selectin and Intercellular Adhesion Molecule-1 Differs Between Crohn's Disease and Ulcerative Colitis"
- DIALOG(R) FILE 144: PASCAL, ACCESSION NUMBER 04065741, XP002001919 & BRIT.MED.J., no. 5936, 1974, pages 83-84, F.J.T.WACKERS ET AL.: "NECROTIZING VASCULITIS AND ULCERATIVE COLITIS"
- ARTHRITIS & RHEUMATISM, vol. 38, no. 9SUP, 21 - 26 Octobre 1995, page S169 XP000569609 C.O'CONNOR ET AL.: "PREVENTION OF RECURRENT THROMBOSIS IN ANTIPHOSPHOLIPID SYNDROME (APS)"

## Description

La présente invention a pour objet l'utilisation de la sulfasalazine ou de son métabolite, l'acide 5-aminosalicylique (sous forme de monomère ou de dimère) pour la préparation d'un médicament utile dans le traitement de l'insuffisance veineuse, dans le traitement curatif et le maintien de l'état de rémission lors d'ulcères veineux.

La sulfasalazine et son métabolite actif l'acide 5-aminosalicylique (5-ASA) sont utilisés à peu près exclusivement depuis cinquante ans dans le traitement et le maintien de l'état de rémission de la recto-colite hémorragique et de la maladie de Crohn. Les causes de ces maladies ne sont toujours pas déterminées, mais certains facteurs contribuant à la pathologie sont néanmoins reconnus.
Ainsi la recto-colite hémorragique est caractérisée par une activation des monocytes conduisant à une augmentation de la libération de Tumor Necrosis Factor α (TNFα), une augmentation des taux de leucotriènes, une activation des leucocytes polymorphonucléaires (PMNs), une libération des radicaux libres et une vasoconstriction post-capillaire (Casellas et coll., *Clin. Sci.,* 87*,* 453-458, 1994 ; Uchida et coll., *Clin. Biochem.,* 27, 259-264, 1994, "Inflammatory bowel disease" J.B. Kirsner, Ed., Lea & Febiger, Philadelphia, 1988). De plus, une thrombose locale et un infarctus tissulaire ont été proposés comme facteurs contribuant à la maladie de Crohn (Wakefield et coll. *Lancet,* ii*,* 1057-1062, 1989).

Les mécanismes d'action de la sulfasalazine et du 5-ASA dans le traitement de la recto-colite hémorragique ne sont toujours pas élucidés. La sulfasalazine et le 5-ASA inhibent la synthèse des peptido-leucotriènes (Peskar et coll. *Agents and Actions,* 18, 381-383, 1986 ; Berry et coll., *Br. J*. *Pharmacol.,* 93, 141P, 1988). La sulfasalazine inhibe la 15-hydroxy-prostaglandine déshydrogènase (Berry et coll., *Biochem. Pharmacol.,* 32, 2863-2871, 1983). La sulfasalazine et le 5-ASA sont des capteurs des radicaux libres (Ronne et coll., *Gastroenterology,* 98*,* 1162-1169, 1990), inhibent l'activation des neutrophiles *in vitro* (Molin et Stendhal, *Acta. Med. Scand.,* 206, 451-457, 1979) et la libération des cytokines par les monocytes (Lamming et coll., *Gastroenterology,* 96, A525, 1989). Récemment on a montré que la sulfasalazine induit *in vitro* dans des cellules endotheliales de veine ombilicale humaine en culture, une diminution de l'expression de la E-selectin et de la molécule-1 d'adhésion intercellulaire (ICAM-1) (Pooley et coll., *Dig. Dis. Sci.,* 40, 219-225, 1995).

L'hypertension veineuse chronique associée à l'insuffisance veineuse a des répercussions sur le fonctionnement de la microcirculation cutanée. Les globules blancs notamment les monocytes et les PMNs traversent le réseau capillaire plus lentement et sont activés (Butler et Coleridge Smith, *J*. *Dermatol. Surg. Oncol.,* 20, 474-480, 1994). Cette activation provoque l'adhésion de ces cellules sur la paroi des veinules post-capillaires et la libération des médiateurs de l'inflammation qui peuvent contribuer à la destruction tissulaire associée à l'ulcère.
L'insuffisance veineuse est souvent la conséquence d'une thrombose veineuse. De plus des essais cliniques avec la pentoxyfylline qui inhibe l'activation des neutrophiles se sont montrés prometteurs pour accélérer le traitement des patients souffrant d'ulcères veineux.

L'utilisation du 5-ASA en application topique pour le traitement curatif d'ulcères des jambes a été décrit (EP-A-0352826), ainsi que l'utilisation de la sulfasalazine, en association avec d'autres médicaments, dans le traitement de phlébites (Wackers et al., Brit. Med. J. no. 5936, 1974, pp. 83-84)

La demanderesse a mis en évidence pour la première fois l'effet de la sulfasalazine sur l'enroulement et l'adhésion des globules blancs *in vivo* dans les veinules post-capillaires mésentériques chez le rat.
Les résultas obtenus dans ce test montrent que l'application topique de sulfasalazine à la vasculature mésentérique chez le rat entraîne une diminution dose-dépendante de l'activation des globules blancs induite par la f-met-leu-phe. Cette inhibition est significative à partir d'une concentration en sulfasalazine de 1 mM.

Ainsi la sulfasalazine et/ou son métabolite actif le 5-ASA peuvent être utilisés pour la préparation d'un médicament utile dans le traitement de l'insuffisance veineuse, le traitement curatif et le maintien de l'état de rémission lors d'ulcères veineux.

A cet effet les composés peuvent être présentés sous toutes formes pharmaceutiques adaptées à l'administration orale et à l'application topique, en association avec des excipients appropriés. Ces formes sont dosées pour permettre une administration journalière de 0,1 à 4 g de substance active.

## Revendications

1. Utilisation de la sulfasalazine ou de l'acide 5-aminosalicylique (sous forme de monomère ou de dimère) pour la fabrication d'un médicament utile dans le traitement de l'insuffisance veineuse.

2. Utilisation de la sulfasalazine ou de l'acide 5-aminosalicylique (sous forme de monomère ou de dimère) pour la fabrication d'un médicament utile dans le maintien de l'état de rémission des ulcères veineux.

3. Utilisation selon l'une quelconque des revendications 1 et 2 caractérisée en ce que le médicament est administrable par voie orale.

4. Utilisation selon l'une quelconque des revendications 1 et 2 caractérisée en ce que le médicament est administrable par voie topique.

5. Utilisation de la sulfasalazine ou de l'acide 5-aminosalicylique (sous forme de monomère ou de dimère) pour la fabrication d'un médicament utile dans le traitement curatif des ulcères veineux le dit médicament étant administrable par voie orale.

## Claims

1. Use of sulphasalazine or 5-aminosalicylic acid (as monomer or dimer) for the manufacture of a medicinal product intended for the treatment of veinous insufficiency.

2. Use of sulphasalazine or 5-aminosalicylic acid (as monomer or dimer) for the manufacture of a medicinal product intended for maintaining the remission state from venous ulcers.

3. Use according to claim 1 or 2 wherein the medicinal product is administered by the oral route.

4. Use according to claim 1 or 2 wherein the medicinal product is administered by the topical route.

5. Use of sulphasalazine or 5-aminosalicylic acid (as monomer or dimer) for the manufacture of a medicinal product intended for the curative treatment of venous ulcers wherein the medicinal product is administered by the oral route.

## Patentansprüche

1. Verwendung von Sulfasalazin oder der 5-Aminosalicylsäure (in Form des Monomeren oder des Dimeren) für die Herstellung eines Arzneimittels zur Behandlung von Veneninsuffizienz.

2. Verwendung von Sulfasalazin oder der 5-Aminosalicylsäure (in Form des Monomeren oder des Dimeren) für die Herstellung eines Arzneimittels zur Aufrechterhaltung des Remissionszustandes von Venengeschwüren.

3. Verwendung nach einem der Ansprüche 1 und 2, dadurch gekennzeichnet, daß das Arzneimittel auf oralem Wege verabreichbar ist.

4. Verwendung nach einem der Ansprüche 1 und 2, dadurch gekennzeichnet, daß das Arzneimittel auf topischem Wege verabreichbar ist.

5. Verwendung von Sulfasalazin oder der 5-Aminosalicylsäure (in Form des Monomeren oder des Dimeren) für die Herstellung eines Arzneimittels zur heilenden Behandlung von Venengeschwüren, welches Arzneimittel auf oralem Wege verabreichbar ist.
